# EUROPÄISCHE PATENTANMELDUNG

(11) **EP 1 010 397 A1**
(43) Veröffentlichungstag der Anmeldung: **21.06.2000**
(21) Anmeldenummer: 99123753.8
(22) Anmeldetag: 30.11.1999
(51) Int. Cl.: A61B 17/12

(54) **Endoskopisches Instrument mit Fadenschlinge**

(30) Priorität: 16.12.1998 AT 83298 U
(71) Anmelder: AMI (Agency for medical innovations GmbH), 6840 GötzisAT (AT)
(72) Erfinder: Höfle, Dipl.Ing.Siegfried, A-6840 Götzis (AT)
(74) Vertreter: Riebling, Peter, Dr.-Ing.

(57) **Zusammenfassung**

Die Erfindung betrifft ein endoskopisches Instrument mit Fadenschlinge, die an ihrem vorderen Ende einen sich selbst zuziehenden Knoten aufweist, welcher so beschaffen ist, daß bei Zug auf das hintere Ende des Fadens dieser sich an der Hülse im Gabelteil zusammenzieht und die Fadenschlinge außer Eingriff mit den Fixierelementen kommt. Das endoskopische Instrument besteht aus einem Gabelteil, welches mindestens eine elastisch federnde und zusammenfaltbare Gabel aufweist, an deren Innenumfang Fixierelemente angeordnet sind, in denen lösbar die Fadenschlinge gehalten ist, welche durch eine im Gabelteil angeordnete Hülse hindurch geführt ist. Das Fixierelement kann über Klemmrillen, über einen Klebstoff, oder über Einhängeösen die Fadenschlinge lösbar fixieren. Durch das erfindungsgemäße endoskopische Instrument kann die Fadenschlinge bei definierter Formgebung zuverlässig geöffnet und geschlossen werden.

## Beschreibung

Die Erfindung betrifft ein endoskopisches Instrument mit Fadenschlinge nach dem Oberbegriff des Schutzanspruches 1. Derartige endoskopische Instrumente mit Bildung einer Fadenschlinge zwecks Abklemmung von Gefäßteilen bei mikrochirurgischen Operationen sind bekannt. Hierzu ist es bekannt, aus einem Applikationsrohr eine Fadenschlinge aus Kunststoff herauszuführen, die an ihrem vorderen Ende einen sich selbst zuziehenden Knoten aufweist. Die Fadenschlinge ist also fertig geknüpft und sobald am hinteren Ende des Fadens, der aus dem Applikationsrohr herausschaut, gezogen wird, wird damit die Fadenschlinge mit ihrem Knoten gegen die vordere Stirnseite des Applikationsrohres geführt und wird zusammengeschoben.

Derartige endoskopische Instrumente mit freibeweglicher Fadenschlinge haben jedoch den Nachteil, daß während des Zusammenziehens der Fadenschlinge der Faden - der bevorzugt aus einem Kunststoffaden besteht - 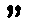 hüpft". Das heißt, es kann keine ruhige, feststehende Fadenschlinge gebildet werden, denn beim Zusammenziehen der Fadenschlinge schlägt diese, bewegt sich an dem abzuklemmenden Gefäß herauf und herunter und verhindert ein sicheres anlegen der Fadenschlingen an einem definierten Gebiet des abzuklemmenden Gefäßes.

Weiterer Nachteil ist, daß die Fadenschlinge frei geführt ist, d. h. sie ist nirgends abgestützt. Aus diesem Grunde fehlt es an einer definierten Formgebung der Fadenschlinge. Die Formgebung ergibt sich nur durch die Steifigkeit des Kunststoffadens. Ist diese jedoch verbogen, verdrillt oder in anderer Weise unbeabsichtigt verfomit, dann verformt sich auch die Fadenschlinge, was zu großen Schwierigkeiten während der endoskopischen Operation führen kann. Es kommt sogar vor, daß in diesen Fällen mit einem zweiten Instrument die Fadenschlinge erst geöffnet werden muß, um sie überhaupt über ein abzuklemmendes Gefäß herüber zu bringen.

Der Erfindung liegt die Aufgabe zugrunde, ein derartiges endoskopisches Instrument mit Bildung einer Fadenschlinge so weiter zu bilden, daß die Fadenschlinge zuverlässig geöffnet werden kann, und daß sie eine definierte Formgebung sowohl bei der Öffnung als auch beim Schließen der Fadenschlinge, zwecks Abklemmung eines Gefäßes behält.

Zur Lösung der gestellten Aufgabe ist die Erfindung durch die technische Lehre des Anspruches 1 gekennzeichnet.

Wesentliches Merkmal der Erfindung ist, daß das endoskopische Instrument aus einem Gabelteil besteht, welches mindestens eine elastisch federnde Gabel aufweist, an deren Innenumfang Fixierelemente angeordnet sind, in denen lösbar die Fadenschlinge gehalten ist, welche durch eine im Gabelteil angeordnete Hülse hindurch geführt ist.

Mit der gegebenen technischen Lehre ergibt sich wesentliche Vorteil, daß nun aufgrund der Anordnung mindestens einer Gabel an einem Gabelteil eine definierte Ausbreitung der Fadenschlinge dadurch erfolgt, daß die Fadenschlinge an Fixierelementen lösbar gehalten ist, die an der Gabel angeordnet sind.

In einer ersten bevorzugten Ausführungsform ist das Fixierelement mit Klemmrillen ausgebildet, welche Klemmrillen klemmend den jeweiligen Teil der Fadenschlinge an dieser Stelle aufnehmen.

In einer anderen Ausgestaltung kann es vorgesehen sein, daß die Fadenschlinge in dem Fixierelement mit einem leicht lösbaren Kleber fixiert ist.

In einer dritten Ausgestaltung kann es vorgesehen sein, daß Einhängeösen vorhanden sind, welches sich bei einem entsprechenden Zug auf die Fadenschlinge selbsttätig öffnen und den Faden damit frei geben.

Wichtig bei allen Ausführungsbeispielen ist, daß nun eine definierte Formgebung der Fadenschlinge im Bereich des Gabelteils gegeben ist, weil ja die Fadenschlinge zwischen den Fixierelementen, die möglichst gleichmäßig verteilt an der Gabel angeordnet sind, aufgespannt ist.

Nun ist es erstmals möglich, in genau definierter Lage ein Gefäß abzuklemmen, denn das gesamte endoskopische Instrument mit der zwischen dem Gabelteil aufgespannten Fadenschlinge wird über das abzuklemmende Gefäß gebracht, ohne daß die Gefahr besteht, daß die Fadenschlinge sich verwirbelt, verbiegt oder verkantet. Sobald die Fadenschlinge an dem richtigen Ort der Abklemmung sich befindet, wird ein Zug auf das hintere Ende des Fadens ausgeübt, wodurch sich der Knoten an der Hülse im Gabelteil zusammenzieht, die Fadenschlinge außer Eingriff mit den Fixierelementen kommt und nun das Gefäß in gewünschter Weise abklemmt.

Es wird dann das Instrument mit seiner Hülse von dem gebildeten Knoten etwas nach hinten gezogen, so daß die beiden hinteren Fadenenden frei werden, wo dann abgeschnitten wird.

In der Einleitung wurde bereits schon darauf hingewiesen, daß es mehrere Ausführungsformen für die Ausbreitung einer derartigen Fadenschlinge gibt. Wichtig bei allen Ausführungsformen ist, daß die Gabel so gestaltet ist, daß sie zusammenfaltbar ist. Geht man von zwei symmetrisch zueinander ausgebildeten Gabeln aus, dann ist Voraussetzung für diese Ausführungsform, daß die Gabeln flexibel im Gabelteil gehalten sind, z. B. sind sie aus einem leicht biegbaren Kunststoff ausgebildet. Beide Gabeln sind leicht zusammendrückbar, d. h. bei einem in das Applikationsrohr eingefahrenen Instrument liegen die beiden Gabelteile dicht aneinander an und zwischen diesen Gabelteilen ist die Fadenschlinge bereits schon gebildet.

Erst wenn das Instrument aus dem Applikationsrohr herausgefahren wird, schnellen die Gabeln unter Aufwendung einer elastischen Rückstellkraft nach außen auf erreichen ihre endgültige, auseinander gespreizte Formgebung und spannen zwischen sich die Fadenschlinge auf.

Bei einer anderen Ausgestaltung, wo nur eine einzige Gabel vorhanden ist, ist hierbei Voraussetzung, daß die Gabel in sich gestreckt ( d. h. also in Längsrichtung ausgebreitet) in dem Applikationsrohr liegt, wobei ebenfalls die Fadenschlinge bereits schon dort aufgespannt ist. Sobald diese Einzelgabel aus dem Applikationsrohr heraustritt, entfaltet sie sich aufgrund ihres elastischen Rückstellvermögens zu einem etwa halbrunden, oder runden oder sichelförmigen Körper, der zwischen sich ebenfalls die Fadenschlinge aufspannt.

In einer dritten Ausgestaltung kann es vorgesehen sein, daß am Gabelteil zwar zwei Gabeln ausgebildet sind, die zueinander spiegelsymmetrisch sind, die Gabeln sind aber in ihrem vorderen Bereich miteinander verbunden und weisen dort eine Schwachstelle auf Für diese Ausführungsform ist es ebenfalls Voraussetzung, daß die beiden Gabeln in eingefahrenem Zustand dicht aneinander liegen, daß sie aber aufgrund ihres elastischen Rückstellvermögens auseinander schnellen und zwischen sich die Fadenschlinge ausbilden.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung, offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technik neu sind.

Im folgenden wird die Erfindung anhand von lediglich einem Ausführungsweg der darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: Perspektivische Ansicht eines Instrumentes nach der Erfindung
- Figur 2:: Schnitt durch ein Instrument nach der Erfindung
- Figur 3:: Die um 90° gedrehte Schnittansicht gemäß Fig. 2
- Figur 4:: Eine zweite Ausführungsform für ein derartiges Instrument mit einer einzigen Gabel
- Figur 5:: Schematisiert eine dritte Ausführungsform mit einer durchgehenden Gabel
- Figur 6:: Die perspektivische Ansicht einer Zugstange
- Figur 7:: Eine Einzelheit gemäß Figur 6 der Zugstange
- Figur 8:: Schnitt durch die Zugstange nach Figur 6
- Figur 9:: um 90° gedrehte Ansicht gegenüber Figur 8

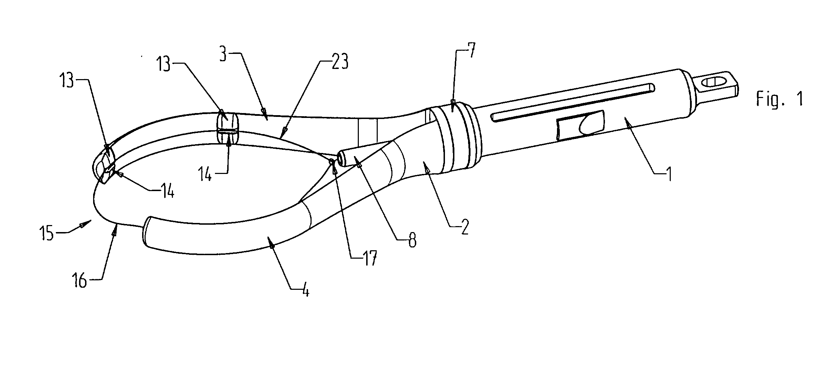

Das Instrument besteht im wesentlichen aus einem Halterungsschaft 1, an dem eine Rastnase ausgebildet ist, so daß das Instrument mit diesem Halterungsschaft 1 in die vordere Aufnahmeöffnung eines ansich bekannten Applikatorrohres eines endoskopischen Applikators einsteckbar und dort leicht lösbar verrastbar ist. Der Halterungsschaft 1 geht über in ein Gabelteil 2, an dem werkstoffeinstückig zwei Gabeln 3, 4 angeformt sind, welche mindestens in ihrem Befestigungsbereich am Gabelteil 2 aufgrund ihres elastischen Rückstellvermögens elastisch ausgebildet sind und in radialer Richtung auswärts ein Federungsvermögen aufweisen. In Figur 1 ist das Instrument in seiner gestreckten Gebrauchslage dargestellt. Es ist erkennbar, daß zwischen den Gabeln 3, 4 eine Fadenschlinge 16 aufgespannt ist, welche zwischen Fixierelementen 13 aufgenommen ist. Derartige Fixierelemente 13 sind in regelmäßigen Abständen am Innenumfang der Gabeln 3, 4 angeordnet und jedes Fixierelement weist eine Klemmrille 14 auf weiche in der Lage ist, die jeweilige Fadenschlinge 16 klemmend festzuhalten. Die Fadenschlinge 16 läuft also in den Klemmrillen 14 um, und bildet vor einer Hülse 8 einen Knoten 17.

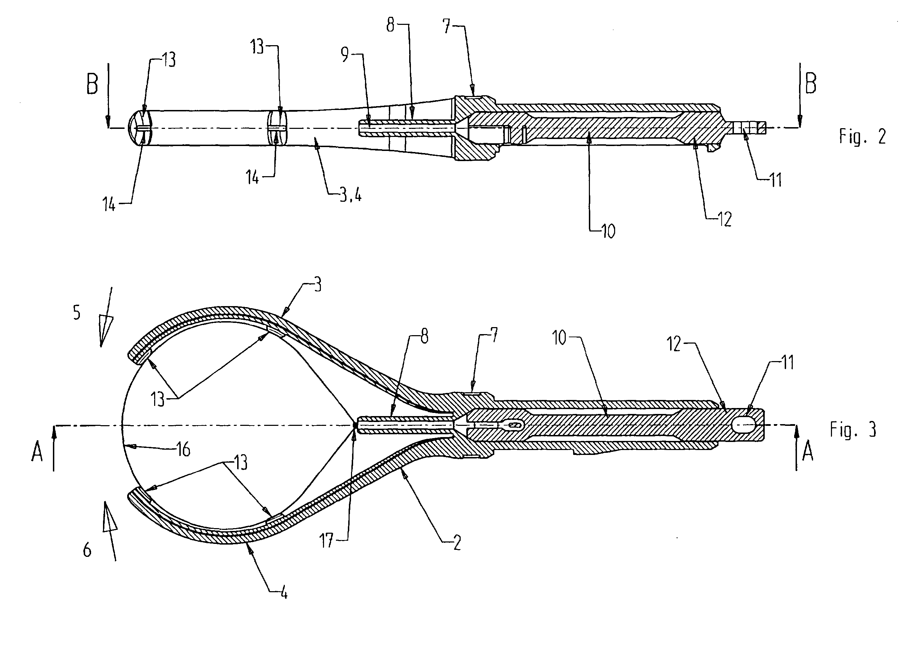

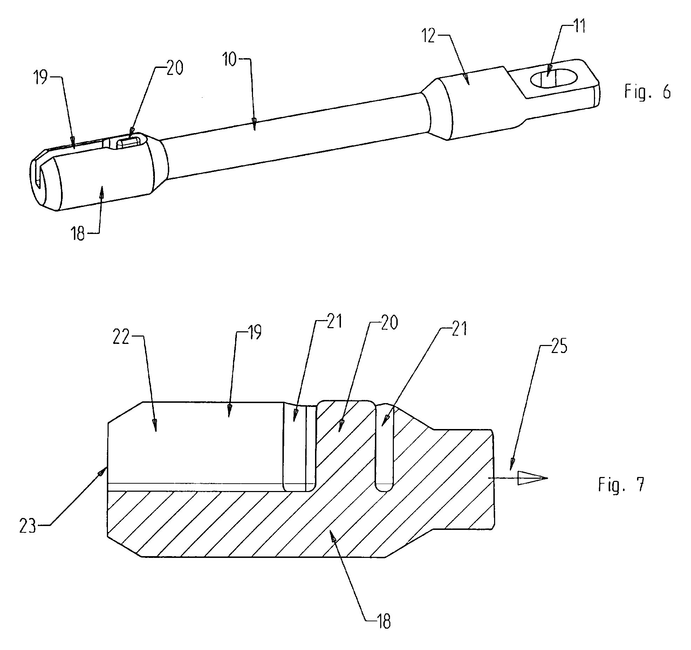

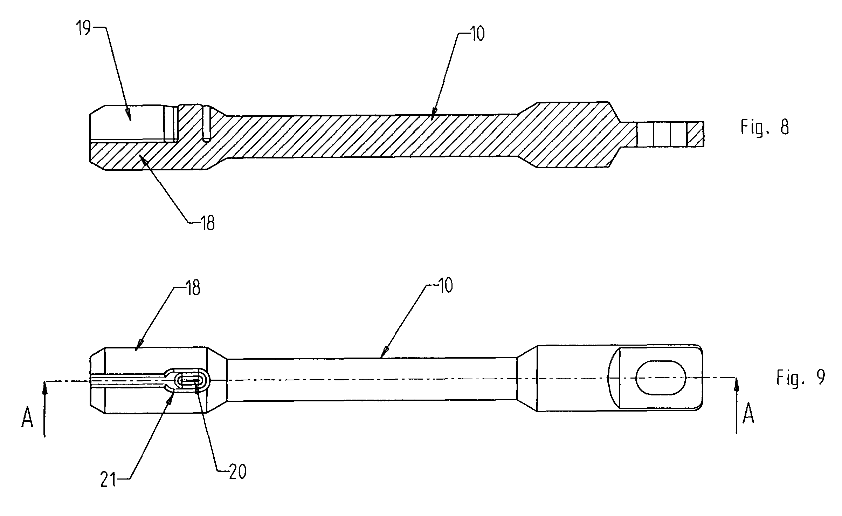

Die Figuren 2 und 3 zeigen weitere Einzelheiten des Instrumentes nach Figur 1. Es ist erkennbar, daß am Gabelteil 2 eine auf dem Innenraum der Gabeln 3, 4 einwärts gerichtete Hülse 8 angeformt ist, welche eine Bohrung 9 ausbildet, durch welche der Faden 23 hindurch geführt ist. Das Ende des Fadens 23 (Fadenende 22) ist hierbei in einer Zugstange 10 gesichert, wie es anhand der Figuren 6 - 9 noch näher erläutert werden wird.

Die Zugstange 10 ist längsverschiebbar in dem Halterungsschaft 1 gehalten und wird mittels einer nicht näher dargestellten Zugstange eines endoskopischen Applikators betätigt. Hierbei weist diese Zugstange einen Haken auf der in eine zugeordnete Öse 11 an einer Verdickung 12 der Zugstange 10 eingreift, sodaß diese in axialer Richtung in der Aufnahme des Haltungsschaftes 1 bewegbar ist.

An dem Gabelteil 2 ist noch röntgendichter Ring 7 eingerastet, der ein Aufspüren des Instrumentes im Körper unter Röntgenkontrolle erlaubt, falls wieder Erwarten ein solches Instrument im Körper verloren geht.

Die beiden Gabeln 3, 4 sind in ihrem vorderen Bereich unterbrochen, d. h. sie bilden ein Offenteil 15, welches von einem Teil der Fadenschlinge 16 überbrückt wird.

Die Figur 5 zeigt, daß dieses Offenteil 15 nicht lösungsnotwendig ist. Es ist dargestellt, daß die Gabeln 3, 4 werkstoffeinstückig miteinander verbunden sind und hierbei die Gabel 3b bilden, welche durchgehend ausgebildet ist. Anstelle des Offenteils 15 ist hierbei eine Schwachstelle 24 in diesem Gabelkörper der Gabeln 3b angeordnet, so daß diese ebenfalls in zusammengefaltetem Zustand in den Pfeilrichtungen 5, 6 elastisch federnd einwärts verformbar sind.

Gleiches gilt im übrigen auch für die Gabeln 3, 4 nach Figur 1, die ebenfalls in zusammengeschobenem Zustand in den Pfeilrichtungen 5, 6 eng aneinander liegend zusammengefaltet sind.

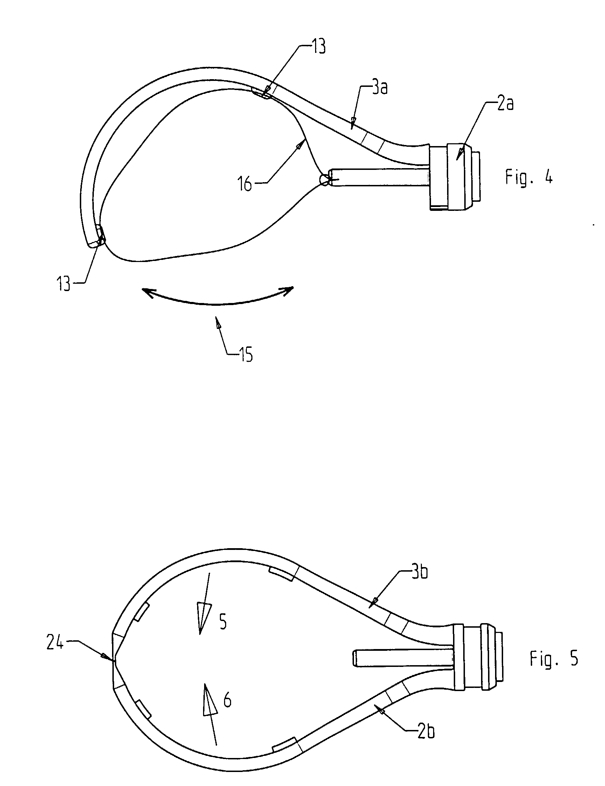

Die Figur 4 zeigt als weiteres Ausführungsbeispiel, daß eine einseitig offene Gabel 3a an einem Gabelteil 2a vorgesehen werden kann, wobei die Gabel 3a z. B. einen Winkel von 200° oder 260° überstreichen kann, während dann eine Offenstelle 15 an der Seite angeordnet ist. Auch zwischen einer derartigen asymmetrischen Gabel kann eine derartige Fadenschlinge 16 im Bereich von Fixierelementen 13 aufgespannt werden.

Dieser Gabelteil 3a würde sich dann in Längsrichtung strecken, wenn das gesamte Instrument in ein zylindrische Ausnehmung eines Applikatorrohres hineingezogen wird.

Die Zugstange 10 nach Figur 1 dient zur Beaufschlagung des Fadens 23 mit einer entsprechenden Zugbewegung. Hierbei ist gemäß Figur 6 am vorderen Ende der Zugstange ein Führungselement 18 angeformt, welches eine Längsnut 19 aufweist, die von einem radial auswärts gerichteten, freistehenden Zapfen 20 abgeschlossen wird, um den herum eine Ringnut 21 ausgebildet ist.

Das zu sichernde Fadenende 22 wird daher durch die Längsnut 19 hindurch in die Ringnut 21 eingeführt, und um den Zapfen 20 herumgeschlungen, so daß es gegen Zugkräfte gesichert festgelegt ist.

Wird nun ein entsprechender Zug auf die Zugstange 10 in Pfeilrichtung 25 ausgeübt, dann wird der Faden 23 entsprechend unter Zugspannung gesetzt und die Fadenschlinge 16 gerät unter Zugspannung, wodurch die Fadenschlinge außer Eingriff mit den Klemmrillen 14 im Bereich der Fixierelemente 13 kommt.

Mit weiterem Zug auf das Zugelement kommt der Knoten 17 zum Anschlag an der Stirnseite der Hülse 8, wodurch sich die Fadenschlinge jetzt um das abzuklemmende Gefäß immer enger zuzieht und schließlich das Gefäß abklemmt. Es wird dann das gesamte Instrument etwas nach hinten gezogen, so daß der Knoten 17 von der Stirnseite der Hülse 8 freikommt und in diesem Bereich wird dann der Faden abgeschnitten.

Es kann dann das gesamte Instrument in das Applikatorrohr eingefahren werden, wodurch sich die Gabeln in Pfeilrichtungen 5, 6 gegeneinander bewegen und zusammenlegen, so daß das gesamte Instrument in das Applikatorrohr zusammengefaltet eingefahren wird.

Vorteil dieses Instrumentes ist also, daß eine definierte Fadenschlinge, definierter Formgebung um ein abzuklemmendes Gefäß herumgelegt werden kann und dann zugezogen werden kann, ohne daß die Fadenschlinge sich um das Gefäß herumbewegt, schlägt oder verkantet.

### Zeichnungslegende

- 1.: Halterungsschaft
- 2.: Gabelteil 2a, 2b
- 3.: Gabel 3a, 3b
- 4.: Gabel
- 5.: Pfeilrichtung
- 6.: Pfeilrichtung
- 7.: Ring
- 8.: Hülse
- 9.: Bohrung
- 10.: Zugstange
- 11.: Öse
- 12.: Verdickung
- 13.: Fixierelement
- 14.: Klemmrillen
- 15.: Offenteil
- 16.: Fadenschlinge
- 17.: Knoten
- 18.: Führungselement
- 19.: Längsnut
- 20.: Zapfen
- 21.: Ringnut
- 22.: Fadenende
- 23.: Faden
- 24.: Schwachstelle
- 25.: Pfeilrichtung

## Patentansprüche

1. Endoskopisches Instrument mit Fadenschlinge, die an ihrem vorderen Ende einen sich selbst zuziehenden Knoten aufweist, wobei das endoskopische Instrument aus einem Gabelteil besteht, welches mindestens eine elastisch federnde Gabel aufweist, **dadurch gekennzeichnet,** daß verteilt am Innenumfang der Gabel (3; 4) Fixierelemente (13) angeordnet sind, in denen lösbar die Fadenschlinge (16) gehalten ist, welche Fadenschlinge (16) durch eine im Gabelteil (2) angeordnete Hülse (8) hindurch geführt ist, wobei jedes Fixierelement (13) mit Klemmrillen (14) ausgebildet ist, die den jeweiligen Teil der Fadenschlinge (16) an dieser Stelle klemmend aufnehmen.

2. Endoskopisches Instrument mit Fadenschlinge nach Anspruch 1, **dadurch gekennzeichnet,** daß die Fadenschlinge (16) in dem Fixierelement (13) mit einem leicht lösbaren Kleber fixiert ist.

3. Endoskopisches Instrument mit Fadenschlinge nach Anspruch 1, **dadurch gekennzeichnet,** daß am Innenumfang der Gabel (3, 4) Ein hängeösen vorhanden sind, welche sich bei einem entsprechenden Zug auf die Fadenschlinge (16) selbsttätig öffnen und den Faden (23) damit frei geben.

4. Endoskopisches Instrument mit Fadenschlinge nach Anspruch 1 - 3, **dadurch gekennzeichnet,** daß die Fixierelemente (13) gleichmäßig verteilt am Innenumfang der Gabel (3, 4) angeordnet sind.

5. Endoskopisches Instrument mit Fadenschlinge nach Anspruch 1 - 4, **dadurch gekennzeichnet,** daß der Knoten (17) so beschaffen ist, daß bei Zug auf das hintere Ende des Fadens (23) sich der Knoten (17) an der Hülse (8) im Gabelteil (2) zusammenzieht und die Fadenschlinge (16) außer Eingriff mit den Fixierelementen (13) kommt.

6. Endoskopisches Instrument mit Fadenschlinge nach Anspruch 1 - 5, **dadurch gekennzeichnet,** daß die Gabel (3, 4) im wesentlichen elastisch federnd zusammenfaltbar ausgebildet ist.

7. Endoskopisches Instrument mit Fadenschlinge nach Anspruch 6, **dadurch gekennzeichnet,** daß die Gabel (3, 4) aus einem leicht biegbaren Kunststoff ausgebildet ist.

8. Endoskopisches Instrument mit Fadenschlinge nach Anspruch 6 oder 7, **dadurch gekennzeichnet,** daß die Gabel (3, 4) aus ihrer ursprünglichen, etwa halbrunden, runden oder sichelförmigen Form in sich gestreckt werden kann.

9. Endoskopisches Instrument mit Fadenschlinge nach Anspruch 6 - 8, **dadurch gekennzeichnet,** daß die Gabel (3, 4) flexibel im Gabelteil (2) gehalten ist.

10. Endoskopisches Instrument mit Fadenschlinge nach Anspruch 1 - 9, **dadurch gekennzeichnet,** daß bei zwei symmetrisch zueinander ausgebildeten Gabeln (3b) diese in ihrem vorderen Bereich über eine Schwachstelle (24) miteinander verbundenen sind.

11. Endoskopisches Instrument mit Fadenschlinge nach Anspruch 1 - 10, **dadurch gekennzeichnet,** daß das Instrument zusammengefaltet elastisch federnd im Applikationsrohr untergebracht werden kann und sich beim Ausfahren entfaltet.
